# EUROPEAN PATENT SPECIFICATION

(11) **EP 3 480 222 B1**
(45) Date of publication and mention of the grant of the patent: **22.12.2021**
(21) Application number: 18203896.8
(22) Date of filing: 31.10.2018
(51) Int. Cl.: C08F 212/08, C08F 212/32, C08F 226/06, C08F 220/18, C09D 125/08

(54) **ADDITION POLYMERS FROM NITROGEN HETEROCYCLE CONTAINING MONOMERS AND VINYL ARYLCYCLOBUTENE-CONTAINING MONOMERS**
ADDITIONSPOLYMERE AUS STICKSTOFFHETEROCYCLEN-HALTIGEN MONOMEREN UND VINYLARYLCYCLOBUTEN-HALTIGEN MONOMEREN
POLYMÈRES D'ADDITION DE MONOMÈRES CONTENANT UN HÉTÉROCYCLE D'AZOTE ET DE MONOMÈRES CONTENANT DE L'ARYLCYCLOBUTÈNE VINYLE

(30) Priority: 02.11.2017 US 201715801776
(43) Date of publication of application: 08.05.2019
(73) Proprietor: Rohm and Haas Electronic Materials LLC, Marlborough, MA 01752 (US)
(72) Inventor: HAYES, Colin, Marlborough, MA 01752 (US); AOUDE, Tina, Marlborough, MA 01752 (US); BARR, Robert K., Marlborough, MA 01752 (US); FLEMING, David, Marlborough, MA 01752 (US); GALLAGHER, Michael K., Marlborough, MA 01752 (US); RIENER, Michelle, Marlborough, MA 01752 (US)
(74) Representative: Houghton, Mark Phillip

(56) References cited:
- US-A1- 2015 197 594
- US-A1- 2015 279 512
- US-A1- 2016 040 021
- US-B1- 6 361 926
- YING-HUNG SO ET AL: "Styrene 4-vinylbenzocyclobutene copolymer for microelectronic applications", JOURNAL OF POLYMER SCIENCE, PART A: POLYMER CHEMISTRY, vol. 46, no. 8, 1 January 2008 (2008-01-01), pages 2799-2806, XP055316150, US ISSN: 0887-624X, DOI: 10.1002/pola.22613

## Description

The present invention relates generally to the field of polymer materials, and, more particularly, to copolymers of, in copolymerized form, a monomer mixture of one or more aromatic addition polymerizable monomers, one or more nitrogen heterocycle containing addition polymerizable monomers, and one or more addition polymerizable arylcyclobutene-containing monomers A which have an addition polymerizable group, for example, a vinyl group, and one or more alkyl, alkoxy or other electron donating group and which are useful in the manufacture of electronic devices.

Arylcyclobutene-containing polymers are known to be useful as dielectric materials in a variety of electronic applications, such as microelectronic packaging and interconnect applications. More recently, vinyl arylcyclobutene-styrene copolymers have been shown to provide many of the dielectric benefits of a divinyl siloxane bis-benzocyclobutane (DVS-BCB) polymer at a significantly reduced cost. So et al., J Poly Sci A, Volume 46, Issue 8, 15 April 2008, pages 2799-2806, discloses blends of a curable copolymer of styrene and vinyl BCB for use in dielectric applications. For example, styrene and 4-vinylbenzocyclobutene (vinyl-BCB) random copolymers were prepared by free radical polymerization and studied for suitability as a dielectric material for microelectronic applications. The polymers had a low dielectric constant. However, the So et al. styrene containing copolymers suffer from low elongation and, thus, form brittle polymer films, making them difficult to process in roll to roll dry films, such as, for example, a dielectric laminate or a printed circuit board (PCB) laminate. In toughened blends with a thermoplastic elastomer at 20 wt % (a star-shaped polystyrene-block-polybutadiene), the polymers exhibited improved fracture toughness and retained thermal stability; the polymer blends are desirable for high frequency-high speed mobile communication applications. The toughened blends required additional toughener which comprised the heat stability of the composition. Accordingly, a more flexible styrenic vinyl arylcyclobutene containing copolymer would be desirable.

The present inventors have sought to solve the problem of providing a heat stable dielectric copolymer material that provides adequate flexibility to enable one to process the materials in roll to roll dry films.

US 2015/0197594 discloses high-chi block copolymers for directed self-assembly.

### STATEMENT OF THE INVENTION

In accordance with a first aspect of the present invention, a polymer composition comprises, in copolymerized form, a monomer mixture of from 10 to 90 wt.% of one or more addition polymerizable arylcyclobutene-containing monomer A; from 5 to 70 wt.% of one or more aromatic addition polymerizable second monomers, chosen from styrene, α-methyl styrene, allyloxystyrene, allyl terminated polyarylene ethers or maleimide terminated polyarylene ethers; and from 5 to 40 wt.% of one or more nitrogen heterocycle containing addition polymerizable third monomers chosen from vinyl pyridine and vinyl imidazole or, preferably, two or more such monomers, and optionally, an addition polymerizable fourth monomer, such as n-butyl acrylate, a fatty (meth)acrylate, or a diene monomer, such as β-myrcene; or mixtures thereof; wherein monomer A has Structure A: 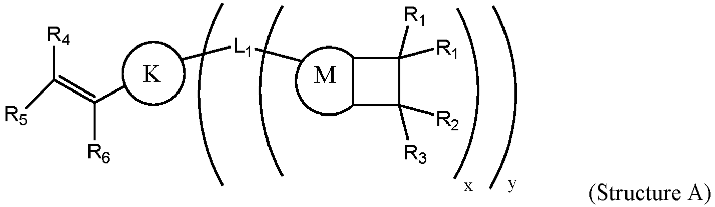 wherein K is a covalent bond or a divalent group chosen from a C₁ to C₆ alkyl substituted or unsubstituted divalent aryl group, a C₁ to C₆ alkyl substituted or unsubstituted divalent heteroaryl group, such as an aryloxy group; a C₁-C₃₀ divalent alkyl group; a C₁-C₃₀ heteroatom containing alkyl group; a divalent C₁-C₃₀ alkylene group, a carbonyl group, an ether group, a thioether group, an ester group, a carboxyl group or a cyano group;
M is a divalent aromatic group chosen from a C₁ to C₆ alkyl substituted or unsubstituted divalent aryl group, a C₁ to C₆ alkyl substituted or unsubstituted divalent heteroaryl group; and
L₁ is selected from a covalent bond or is a hydrocarbon linking group having a valence of x +1, preferably, when x is 1, L₁ is divalent hydrocarbon group, such as an alkylene group or an alkyl substituted alkylene group, a C₁-C₃₀ heteroatom containing hydrocarbon group, or a C₁-C₃₀ substituted heterohydrocarbyl group; and
R₁ through R₆ are each independently selected from a monovalent group chosen from hydrogen, deuterium, halogen, a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group, wherein at least one of R₁, R₂ and R₃ is chosen from a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group; or, preferably, one or more of R₁, R₂ and R₃ is chosen from a C₁ to C₆ 6 alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, an hydroxyl group, an aryl group, C₁ to C₆ alkyl substituted aryl group, a heteroaryl group, or a C₁ to C₆ alkyl substituted heteroaryl group; and,
x and y are each independently an integer from 1 to 5 wherein y is 1 when L₁ is a covalent bond, or, preferably, y is 1 and x is 1 or 2;
wherein all weights based on the total solids weight of monomers used to make the copolymer with all monomer wt.%s adding to 100%.

Preferably, in accordance with the first aspect of the present invention, the polymer composition comprises a polymer of, in copolymerized form, a monomer mixture of the one or more addition polymerizable arylcyclobutene-containing monomers A, the one or more aromatic addition polymerizable second monomers chosen from styrene, α-methyl styrene, β-myrcene, allyloxystyrene, allyl terminated polyarylene ethers or maleimide terminated polyarylene ethers; and the nitrogen heterocycle containing addition polymerizable third monomer.

Preferably, in accordance with the first aspect of the present invention, the polymer composition comprises a polymer of, in copolymerized form, a monomer mixture of the one or more addition polymerizable arylcyclobutene-containing monomers A; the one or more aromatic addition polymerizable second monomers; the one or more nitrogen heterocycle containing addition polymerizable third monomer chosen from a nitrogen heterocycle containing third monomer vinyl monomer, such as N-vinyl pyridine, N-vinyl imidazole or other vinyl pyridine isomers such as 2-vinyl pyridine, or, more preferably, two or more addition polymerizable group containing nitrogen heterocycle containing third monomers, such as N-vinyl pyridine and N-vinyl imidazole.

Preferably, in accordance with the first aspect of the present invention, the polymer composition comprises a polymer of, in copolymerized form, a monomer mixture of the one or more addition polymerizable arylcyclobutene-containing monomers A, the one or more aromatic addition polymerizable second monomers, the one or more addition polymerizable nitrogen heterocycle containing monomers, and one or more addition polymerizable fourth monomers chosen from acrylates or methacrylates, such as cyclohexyl methacrylate, n-butyl methacrylate, cyclohexyl acrylate and fatty (meth)acrylates; maleimides and bis-maleimides; cyclic anhydrides, such as maleic anhydride, 3-methylenedihydrofuran-2,5-dione or itaconic anhydride; linear and branched alkenes, such as hexene; cyclic olefins, such as cyclopentene, cyclohexene or cyclooctene; and fourth monomers containing a second dienophile or addition polymerizable group, such as benzocyclobutene (BCB) containing crosslinkers, for example, vinyl benzocyclobutene, or divinylsiloxyl bis-benzocyclobutene (DVS-BCB); allyl methacrylate; divinyl benzene; dienes, such as, butadiene, cyclopentadiene, β-myrcene, cyclooctadiene, or tetraphenylcyclopentadienone; allyloxystyrene; allyl, or maleimide terminated polyols, or polysiloxanes; or maleimide terminated polyimides; preferably, the fourth monomer comprises n-butyl acrylate, cyclohexyl acrylate, cyclohexyl methacrylate or C₈ to C₂₀ linear alkyl (meth)acrylates, such as fatty (meth)acrylates.

In accordance with the first aspect of the present invention, the addition polymerizable fourth monomers preferably are addition polymerizable oligomers useful to form rubbery or soft polymers, such as dienes, like myrcene or butadiene, n-butyl or C₈ to C₂₀ linear alkyl (meth)acrylates or, more preferably, monomers having a normal boiling point of at least 150 °C. For lower boiling monomers (below 150 °C), such as, C₁ to C₆ alkyl (meth)acrylates, hexene, cyclopentene, cyclohexene, butadiene, and cyclopentadiene, a pressurized reactor can be used to prevent loss through evaporation of the unsaturated compound prior to reaction with the arylcyclobutene-containing monomers A.

In accordance with the first aspect of the present invention, the polymer composition may further comprise a separate crosslinker, such as a bis-BCB monomer, for example, divinylsiloxy bis-benzocyclobutene (DVS-BCB), or a monomer containing two or more dienophile groups, such as divinyl benzene; a curing agent, such as an initiator, a photoinitiator or other photoactive compound; a catalyst, or more than one of these. The polymer composition can be crosslinked via thermally induced or light induced initiation in the presence of the curing agent and a monomer containing two or more dienophile groups which are addition polymerizable, or by ring opening of the arylcyclobutene groups on the polymer in the presence of any monomer containing two or more dienophile groups.

In accordance with the first aspect of the present invention, the polymer composition comprises, in copolymerized form, a monomer mixture of from 10 to 90 wt.% or, preferably, from 15 to 70 wt.% of the one or more addition polymerizable arylcyclobutene-containing monomers A; from 5 to 70 wt.% or, preferably, from 20 to 55 wt.% of the one or more aromatic addition polymerizable second monomer; from 5 to 40 wt.% or, preferably, from 5 to 35 wt.%, or more preferably, from 10 to 35 wt.% of the one or more nitrogen heterocycle containing addition polymerizable third monomers, all weights based on the total solids weight of monomers used to make the copolymer with all monomer wt.%s adding to 100%.

In accordance with the first aspect of the present invention, the polymer composition may comprise, in copolymerized form, a monomer mixture of from 10 to 90 wt.% or, preferably, from 15 to 70 wt.% of the one or more addition polymerizable arylcyclobutene-containing monomers A; from 5 to 70 wt.% or, preferably, from 20 to 55 wt.% of the one or more aromatic addition polymerizable second monomer; from 5 to 40 wt.% or, preferably, from 5 to 35 wt.%, or more preferably, from 10 to 35 wt.% of the one or more nitrogen heterocycle containing addition polymerizable third monomers, and from 5 to 40 wt.% or, preferably, from 10 to 35 wt.% of the one or more addition polymerizable fourth monomers, all weights based on the total solids weight of monomers used to make the copolymer with all monomer wt.%s adding to 100%.

Preferably, in accordance with the first aspect of the present invention, the polymer composition may comprise, in copolymerized form, a monomer mixture of the one or more addition polymerizable arylcyclobutene-containing monomers A, the one or more aromatic addition polymerizable second monomers, and from 5 to 40 wt.%, or, more preferably, from 10 to 40 wt.% or, even more preferably, from 15 to 35 wt.%, in total, of the one or more addition polymerizable nitrogen heterocycle containing third monomers plus the one or more addition polymerizable fourth monomers, based on the total solids weight of the monomers used to make the copolymer with all monomer wt.%s adding to 100%.

In accordance with the first aspect of the present invention, the polymer composition may comprise, in copolymerized form, the monomer mixture of the one or more addition polymerizable arylcyclobutene monomers A, the one or more aromatic addition polymerizable second monomer, the one or more addition polymerizable nitrogen heterocycle containing third monomers, and, if desired, the one or more addition polymerizable fourth monomers, wherein the composition further comprises a crosslinker chosen from a crosslinking monomer, such as monomer containing a second addition polymerizable or dienophile group, and can be crosslinked via thermally induced or light induced initiation in the presence of a curing agent, such as one chosen from a thermal initiator, a photoactive compound or a photoinitiator.

In accordance with the first aspect of the present invention, the polymer composition may comprise, in copolymerized form, the monomer mixture of the one or more addition polymerizable arylcyclobutene monomers A, the one or more aromatic addition polymerizable second monomer, the one or more addition polymerizable nitrogen heterocycle containing third monomers, and, if desired, the one or more addition polymerizable fourth monomers, wherein the composition is curable by ring opening of the arylcyclobutene groups in the polymer and the composition, if desired, contains a crosslinker having two or more arylcyclobutene-containing groups, such as a bis-BCB monomer, for example, divinylsiloxy bis-benzocyclobutene (DVS-BCB).

In accordance with the first aspect of the present invention, the polymer composition may comprise, in copolymerized form, the monomer mixture of the one or more addition polymerizable arylcyclobutene monomers A, the one or more aromatic addition polymerizable second monomer, the one or more addition polymerizable nitrogen heterocycle containing third monomers, and, if desired, the one or more addition polymerizable fourth monomers, wherein the composition is curable by ring opening of the arylcyclobutene groups in the polymer and the composition, if desired, contains a crosslinker having two or more arylcyclobutene-containing groups.

In accordance with the first aspect of the present invention, the polymer composition may comprise, in copolymerized form, the monomer mixture of the one or more addition polymerizable arylcyclobutene monomers A, the one or more aromatic addition polymerizable second monomer, the one or more addition polymerizable nitrogen heterocycle containing third monomers, and, if desired, the one or more addition polymerizable fourth monomers, wherein the polymer has one or more carboxylic acid, alcohol or amide groups and the composition further comprises a condensation crosslinker such as a diamine or a polymeric diol, such as a polyester diol or a polyether diol.

In accordance with the first aspect of the present invention, the polymer composition comprises from 10 to 80 wt.%, or, preferably, from 20 to 50 wt.% of polymer solids, based on the total weight of the polymer and the remainder of a polar aprotic or polar protic organic solvent, such as a polar protic solvent, such as an (cyclo)alkanol or (cyclo)alkanone, or a polar aprotic solvent, such as an alkyl ester, amide or sultone.

In accordance with a second aspect of the present invention, a thin film or coating on a substrate comprises the polymer composition of the first aspect of the present invention of, in copolymerized form, from 10 to 90 wt.% of a monomer mixture of the one or more addition polymerizable arylcyclobutene-containing monomer A; from 5 to 70 wt.% of the one or more aromatic addition polymerizable second monomers chosen from styrene, α-methyl styrene, allyloxystyrene, allyl terminated polyarylene ethers or maleimide terminated polyarylene ethers; and and from 5 to 40 wt.% of the one or more addition polymerizable third monomers chosen from vinyl pyridine and vinyl imidazole; wherein monomer A has the above Structure A; wherein all weights based on the total solids weight of monomers used to make the copolymer with all monomer wt.%s adding to 100%.

In accordance with a second aspect of the present invention, a thin film or coating on a substrate comprises the polymer composition of the first aspect of the present invention of, in copolymerized form, a monomer mixture of the one or more addition polymerizable arylcyclobutene-containing monomer A, the one or more aromatic addition polymerizable second monomers, one or more addition polymerizable nitrogen heterocycle containing monomers, and one or more addition polymerizable fourth monomers, wherein monomer A has the above Structure A.

In accordance with the thin film or coating of the second aspect of the present invention, the polymer composition comprises, in copolymerized form, a monomer mixture of from 10 to 90 wt.% or, preferably, from 15 to 70 wt.% of the one or more addition polymerizable arylcyclobutene-containing monomers A; from 5 to 70 wt.% or, preferably, from 20 to 55 wt.% of the one or more aromatic addition polymerizable second monomer; and from 5 to 40 wt.% or, preferably, from 10 to 35 wt.% of the one or more nitrogen heterocycle containing addition polymerizable third monomers, all weights based on the total solids weight of monomers used to make the copolymer.

In accordance with the thin film or coating of the second aspect of the present invention, the polymer composition may comprise, in copolymerized form, a monomer mixture of from 10 to 90 wt.% or, preferably, from 15 to 70 wt.% of the one or more addition polymerizable arylcyclobutene-containing monomers A; from 5 to 70 wt.% or, preferably, from 20 to 55 wt.% of the one or more aromatic addition polymerizable second monomers; from 5 to 40 wt.% or, preferably, from 10 to 35 wt.% of the one or more nitrogen heterocycle containing addition polymerizable third monomers, and from 5 to 40 wt.% or, preferably, from 10 to 35 wt.% of the one or more addition polymerizable fourth monomers, all weights based on the total solids weight of monomers used to make the copolymer.

Preferably, in accordance with the thin film or coating of the second aspect of the present invention, the polymer composition may comprise, in copolymerized form, a monomer mixture of the one or more addition polymerizable arylcyclobutene-containing monomers A; the one or more aromatic addition polymerizable second monomers; and from 5 to 40 wt.% or, more preferably, from 10 to 35 wt.%, or, even more preferably, from 15 to 35 wt.%, of the one or more nitrogen heterocycle containing addition polymerizable third monomers plus the one or more addition polymerizable group containing fourth monomers, based on the total solids weight of the monomers used to make the copolymer.

In accordance with the thin film or coating of the second aspect of the present invention, the polymer composition may further comprise a condensation crosslinker, a separate crosslinker monomer having two or more arylcyclobutene-containing groups, such as a bis-BCB monomer, for example, divinylsiloxy bis-benzocyclobutene (DVS-BCB), or a monomer containing two or more dienophile groups, such as divinyl benzene, 9,9-bis(4-vinylbenzyl)-9H-fluorene or an addition polymerizable crosslinker monomer containing two or more allyl groups, such as triallylisocyanurate; a curing agent, such as an initiator, a photoinitiator or other photoactive compound; a catalyst, or more than one of these.

In accordance with the thin film or coating of the second aspect of the present invention, the molar ratio of addition polymerizable groups in the one or more crosslinker monomers to the number molar equivalents of arylcyclobutene groups from the monomers A may range from 0.25:1 to 2.0:1, or, preferably, 0.9:1 to 1.5:1.

In accordance with a third aspect of the present invention, an electronic device contains a dielectric layer comprising the thin film or coating of the second aspect of the present invention on a substrate of the present invention, such as a polymer substrate, for example, polyethylene terephthalate or a polyimide.

In accordance with a fourth aspect of the present invention, a method of making a polymer composition comprises providing an organic solvent such as a polar protic solvent or a polar aprotic solvent, and a monomer mixture of one or more one or more addition polymerizable arylcyclobutene-containing monomers A; the one or more aromatic addition polymerizable second monomers, such as styrene, α-methyl styrene, β-myrcene, allyloxystyrene, allyl terminated polyarylene ethers or maleimide terminated polyarylene ethers; and the one or more nitrogen heterocycle containing addition polymerizable third monomers, such as a vinyl pyridine or a vinyl imidazole or, preferably, two or more such monomers, wherein the monomer A has Structure A, above; and an initiator, such as a thermal initiator or a photo-initiator, and polymerizing the monomer mixture. Where a thermal initiator is used, polymerization further comprises heating the monomer mixture to from room temperature or ambient temperature to less than 140 °C or, preferably, from 60 to 125 °C, or more preferably, less than 110 °C.

In accordance with a fourth aspect of the present invention, the polymerizing can be followed by crosslinking or curing the resulting polymer at from ambient temperature to 140 °C for addition crosslinking in the presence of an initiator and at from 160 to 220 °C for ring opening curing, if desired, in the presence of, respectively, a crosslinker monomer containing two or more dienophile groups, preferably, two or more addition polymerizable groups.

The proportions of organic solvent in accordance with the fourth aspect of the present invention are such as to form a polymer composition comprising from 10 to 80 wt.%, or, preferably, from 20 to 50 wt.% of polymer solids, based on the total weight of the polymer and aqueous alkali or organic solvent.

In accordance with the methods of the fourth aspect of the present invention, the monomer mixture or preferred monomer mixture is any that are useful or preferred in the first aspect of the present invention.

In accordance with a fifth aspect of the present invention, methods of making a thin film or coating on a substrate comprises depositing and evaporating or coating on a substrate the polymer composition of the first aspect of the present invention. The polymer compositions can comprise one or more organic solvents. The compositions can, preferably, further comprise a curing agent chosen from a thermal initiator or a light initiator, a photoactive compound, if desired, with a crosslinker monomer containing two or more addition polymerizable groups or a monomer containing two or more dienophile groups, or a condensation crosslinker, such as a diamine or protected diamine.

In accordance with the methods of the fifth aspect of the present invention, the polymer can be heated in the presence of a curing agent, such as an initiator or a photoinitiator, and, if desired, an addition polymerizable crosslinker, at a temperature of from room temperature or ambient temperature to less than 140°C or, preferably, from 60 to 125 °C, or more preferably, less than 110 °C.

In accordance with the methods of the fifth aspect of the present invention, the methods can further comprise ring opening curing the film or coating at a temperature of from 160 to 220 °C or, preferably, from 180 to 210 °C.

The crosslinker or curing agent in accordance with the methods of the fifth aspect of (using the polymer composition of) the present invention can be any one or more of the crosslinkers or curing agents disclosed for use in the polymer composition of the first aspect of the present invention, disclosed above.

In accordance with a sixth aspect of the present invention, methods of using the polymer compositions of the first aspect of the present invention comprise depositing and evaporating or coating on a circuit substrate the polymer composition of the first aspect of the present invention to form a polymer layer, preferably with heating to a temperature of from 60 to 140 °C to effect a soft bake; photo-curing the polymer layer to obtain a photo-cured layer; and thermally curing the photo-cured layer, such as by ring opening at a temperature of from 160 to 220 °C to form an insulation layer.

Unless otherwise indicated, conditions of temperature and pressure are ambient or room temperature (RT) and standard pressure. All ranges recited are inclusive and combinable.

Unless otherwise indicated, any term containing parentheses refers, alternatively, to the whole term as if no parentheses were present and the term without them, and combinations of each alternative. Thus, the term "(meth)acrylate" refers to an acrylate, a methacrylate, or mixtures thereof.

As used herein, all amounts are percent by weight and all ratios are molar ratios, unless otherwise noted.

All numerical ranges are inclusive of the endpoints and combinable in any order, except where it is clear that such numerical ranges are constrained to add up to 100%.

As used herein, the articles "a", "an" and "the" refer to the singular and the plural.

As used herein, the term "alkyl" includes linear, branched and cyclic alkyl. Likewise, "alkenyl" refers to linear, branched and cyclic alkenyl. "Aryl" refers to aromatic carbocycles and aromatic heterocycles.

As used herein, the term "aliphatic" refers to an open-chain carbon-containing moiety, such as alkyl, alkenyl and alkynyl moieties, which may be linear or branched. Also as used herein, the term "alicyclic" refers to a cyclic aliphatic moiety, such as cycloalkyl and cycloalkenyl. Such alicyclic moieties are non-aromatic, but may include one or more carbon-carbon double bonds. "Halo" refers to fluoro, chloro, bromo, and iodo. The term "(meth)acrylate" refers to both methacrylate and acrylate, and likewise the term (meth)acrylamide refers to both methacrylamide and acrylamide.

Unless the context clearly indicates otherwise, by "substituted" alkyl, alkenyl, or alkynyl is meant that one or more hydrogens on the alkyl, alkenyl, or alkynyl is replaced with one or more substituents chosen from halo, hydroxy, C₁₋₁₀ alkoxy, amino, mono- or di-C₁₋₁₀ hydrocarbyl substituted amino, C₅₋₂₀ aryl, and substituted C₅₋₂₀ aryl. Unless the context clearly indicates otherwise, by "substituted" aryl is meant that one or more hydrogens on the aryl is replaced by one or more substituents chosen from halo, hydroxy, C₁₋₁₀ alkyl, C₂₋₁₀ alkenyl, C₂₋₁₀ alkynyl, C₁₋₁₀ alkoxy, amino, mono- or di- C₁₋₁₀ hydrocarbyl substituted amino, C₅₋₂₀ aryl, and substituted C₅₋₂₀ aryl. "Alkyl" refers to an alkane radical, and includes alkane diradicals (alkylene) and higher-radicals. Likewise, the terms "alkenyl", "alkynyl" and "aryl" refer to the corresponding mono-, di- or higher-radicals of an alkene, alkyne and arene, respectively.

As used herein, the term "addition polymerizable group" means any unsaturation functional group that polymerizes via addition polymerization, including vinyl, vinylidene or allyl groupsand any such group having any alkyl, alkoxy, S, N, P, O or Si heteroatom containing hydrocarbon, siloxy, cyano, aryl, alkylaryl, S, N, P, O or Si heteroaryl, carbonyl, carboxyl(ate), aldehyde, diketo, hydroxyl, amine, imine, azo, phosphorus or sulfur containing group as a substituent or component.

As used herein, the term "aromatic organic residue" embraces an organic residue that has only aromatic character, such as phenyl, as well as an organic residue that contains a combination of aromatic and aliphatic moieties.

As used herein, the term "curing" is meant any process, such as addition crosslinking or condensation, that increases the molecular weight of a polymer material or composition through the use of the methods making or using the compositions in accordance with the present invention. "Curable" refers to any polymer material capable of being cured under certain conditions.

As used herein, the term "ASTM" refers to publications of ASTM International, West Conshohocken, PA.

As used herein, the term "complete cure" means that >98% of the residual heat of reaction of a given composition from an original cure profile has been removed from the composition when re-scanned by DSC, as compared to the known heat content of the composition. This is defined as the peak area integrated on the DSC trace, which corresponds to a heat content such as, for example, 200 Joules per gram (J/g). For a composition having a heat content of 200 J/g, a peak that had 100 J/g after a given thermal treatment (e.g. 200 °C for 1hour) would be considered 50% cured.

As used herein, the term "DSC" or "Differential Scanning Calorimetry" refers to a method of measuring polymer cure profiles or exotherms using a Q2000^{™} DSC instrument (TA Instruments, New Castle, DE). DSC was carried out using a sample of isolated uncured polymer (<5 mg) placed in a sealed Tzero^{™} Aluminum hermetic sample pan (TA instruments). The sample pan was then put in the DSC cell along with a control pan and the DSC was then heated from RT to 300 °C at a rate of 10 °C per minute. For B-staging the arylcyclobutene containing polymers of the present invention, the amount of heat (J/g) liberated from a given sample between 200 and 300 °C is defined to be the "residual degree of cure" or residual heat of reaction due to the arylcyclobutene ring opening reaction which has a peak exotherm of 258 °C.

As used herein, the term "formula weight" refers to the molecular weight of a representative formula depicting a given material.

As used herein, the term "hetero" or "heteroatom" when used referring to an organic group means an O, P, N, S or Si atom.

As used herein, the term "normal boiling point" refers to the boiling point of a given liquid in neat form at standard pressure.

As used herein, the term "oligomer" refers to relatively low molecular weight materials such as dimers, trimers, tetramers, pentamers, and hexamers, including B-staged polymerized material, that are capable of further curing or polymerization.

As used herein, the term "solids" refers to any materials that remain a reaction product of the present invention; thus, solids include monomers and non-volatile additives that do not volatilize upon any of B-staging, polymerization and cure. Solids exclude water, ammonia and volatile solvents.

As used herein, the term "stoichiometry" of a reaction mixture refers to the ratio of molar equivalents of unreacted olefin groups or dienophile groups to unreacted arylcyclobutene groups in a given composition.

As used herein, unless otherwise indicated, the term "weight average molecular weight "or "Mw" means that value determined by gel permeation chromatography (GPC) at room temperature using a Waters Alliance High Pressure Liquid Chromatogram (HPLC) (Waters, Milford, MA) equipped with an isocratic pump, an autosampler (Injection volume (100-150 µL) and a Series of 4 Shodex^{™} (8 mm x 30 cm) columns, each filled with a polystyrene divinyl benzene (PS/DVB) gel against a standard calibrated from polystyrene as standards. As used herein, "number average molecular weight "or "Mn" is measured in the same way as weight average molecular weight and represents the median molecular size in a given polymer composition. As used herein, the term "PDI" refers to the ratio of Mw/Mn.

As used herein, the term "wt.%" stands for weight percent.

As used throughout this specification, the following abbreviations shall have the following meanings, unless the context clearly indicates otherwise: °C = degree Celsius; min. = minutes; hr. = hours; g = gram; L = liter; µm = micron = micrometer; nm = nanometer; mm = millimeter; mL = milliliter; MPa = megapascal; Mw = weight average molecular weight; Mn = number average molecular weight; and AMU = atomic mass unit. Unless otherwise noted, "wt.%" refers to percent by weight, based on the total weight of a referenced composition.

The present inventors have found that a polymer composition comprising, in copolymerized form, one or more addition polymerizable arylcyclobutenes, such as α-methyl vinyl benzocyclobutene, one or more addition polymerizable aromatic monomers, and one or more addition polymerizable nitrogen heterocycle containing monomer exhibits superior film flexibility compared to the same polymers without the nitrogen heterocycle containing monomer. Further, the polymers of the present invention can be formed by conventional addition polymerization at temperatures below 140 °C, which is exceptionally low for dielectrics comprising arylcyclobutene-containing groups.

In accordance with the present invention, the polymer composition may comprise, in copolymerized form, a monomer mixture of one or more addition polymerizable arylcyclobutene-containing monomer A, one or more aromatic addition polymerizable second monomers, such as styrene, and one or more nitrogen heterocycle containing addition polymerizable third monomer, such as a vinyl pyridine or a vinyl imidazole; wherein monomer A has Structure A: 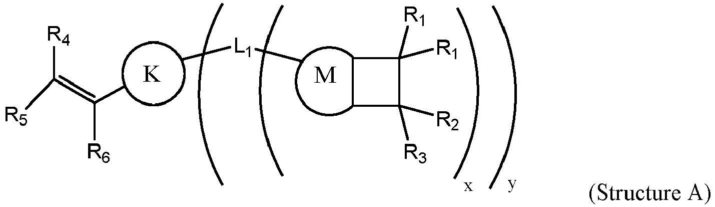 wherein K is a covalent bond or a divalent group chosen from a C₁ to C₆ alkyl substituted or unsubstituted divalent aryl group, a C₁ to C₆ alkyl substituted or unsubstituted divalent heteroaryl group, such as an aryloxy group, a divalent C₁ to C₃₀ alkylene group, a carbonyl group, an ether group, a thioether group, an ester group or a carboxyl group;
M is a divalent aromatic group chosen from a C₁ to C₆ alkyl substituted or unsubstituted divalent aryl group, a C₁ to C₆ alkyl substituted or unsubstituted divalent heteroaryl group;
L₁ is selected from a covalent bond or is a hydrocarbon linking group having a valence of x +1, preferably 2, or L₁ forms with K a C₁₋C₃₀ divalent hydrocarbon group, such as an alkylene group or an alkyl substituted alkylene group, a C₁₋C₃₀ heteroatom containing hydrocarbon group, or a C₁₋C₃₀ substituted heterohydrocarbyl group;
R₁ through R₆ are each independently selected from a monovalent group chosen from hydrogen, deuterium, halogen, a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group, wherein at least one of R₁, R₂ and R₃ is chosen from a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group; or, preferably, one or more of R₁, R₂ and R₃ is chosen from a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, an hydroxyl group, an aryl group, C₁ to C₆ alkyl substituted aryl group, a heteroaryl group, or a C₁ to C₆ alkyl substituted heteroaryl group; and,
x and y are each independently an integer from 1 to 5, or, preferably, y is 1 and x ranges from 1 to 2.

Preferably, the addition polymerizable arylcyclobutene-containing monomer A comprises a vinyl α-C₁ to C₆ alkyl benzocyclobutene, such as a vinyl α-methyl benzocyclobutene (alpha vinyl methyl BCB), α-vinyl ethyl BCB, or α-vmyl propyl BCB.

The arylcyclobutene polymers of the present invention may comprise as polymerized units one addition polymerizable arylcyclobutene-containing monomer A or more than one addition polymerizable arylcyclobutene-containing monomer A.

To insure the flexibility of the polymer composition in accordance with the present invention, suitable amounts addition polymerizable nitrogen heterocycle containing monomer range from 5 to 40 wt.% or, preferably, from 10 to 35 wt.%, based on the total solids weight of the monomer mixture.

To insure the flexibility of the polymer composition in accordance with the present invention, the monomer mixture can comprise, as solids, as little as 3 wt.% or, preferably, as little as 5 wt.% of the addition polymerizable nitrogen heterocycle containing third monomer so long as the total amount of the third monomer and a rubbery fourth monomer, such as n-butyl acrylate, a long chain alkyl (meth)acrylate or butadiene, ranges from at least 10 wt.% or, preferably, at least 12 wt.% of the monomer mixture.

Suitable addition polymerizable fourth monomers for useful in making the polymer compositions in accordance with the present invention are free of arylcyclobutene groups. Suitable fourth monomers can be, for example, (meth)acrylates, such as n-butyl (meth)acrylates and fatty (meth)acrylates, ally group containing monomers, alkenes, dienes and addition polymerizable monomers containing additional functional groups, such as acidic or hydrolyzable groups, and high boiling point (normal boiling point of at least 150 °C) monomers, such as allyl, alkyne or maleimide terminated polyols, polyarylene ethers, or polysiloxanes.

The arylcyclobutene polymer compositions in accordance with the present invention may comprise as polymerized units one or more addition polymerizable fourth monomers comprising a diene, such as butadiene, isoprene, ocimene or β-myrcene; or comprising an additional polymerizable group or a dienophile group, or two or more dienophile groups, such as diallyl ether, allyloxystyrene, divinyl benzene or d iethynyl benzene. Fourth monomers comprising two or more addition polymerizable or dienophile groups may be used to impart addition crosslinking capabilities to the polymer composition of the present invention. Such monomers can also be used as a separate crosslinker in addition to the polymer. The selection of such fourth monomers is within the ability of those skilled in the art.

Suitable addition polymerizable fourth monomers that contain additional functional groups, such as acidic groups, for example, anhydride groups, or other hydrolyzable groups, such as active hydrogen or hydrolyzable sil(ox)ane groups, allow for modular post-polymerization functionalization or curing by condensation. One can, for example, react such monomers with a diamine or multifunctional amine to form an amic acid precursor which can be imidized during a thermal cure process. One can also hydrolyze the anhydride, such as by combining it with aqueous media and alcoholize such monomers, such as with t-butanol, to form monoesters.

In accordance with the methods of making the polymer composition of the present invention, the methods comprise polymerizing the monomer mixture of the present invention via non-controlled free radical addition polymerization using, for example benzoyl peroxide (BPO) or a persulfate or its salt as an initiator. In polymerizing, the monomer mixture is generally heated to from 60 to 140°C, or, preferably, 70 to 125 °C.

Suitable polymerization solvents are any organic solvents which dissolve the one or more monomers and have boiling points above the polymerization temperature of the monomers, such as mesitylene. Exemplary organic solvents include polar aprotic solvents such as amides, esters, such a butyl acetate, and sulfones, and polar protic solvents, such as (cyclo)alkanols.

Polymerization time is typically from 1 to 60 hours, for example, 20 to 45 hours. For certain applications, it may be desired to stop the polymerization at the oligomer stage. Such oligomers composed of one or more monomers of the invention may be composed predominantly of dimers, trimers, or tetramers, and may then be subsequently further polymerized. As used herein, the term "monomer(s) of the present invention" is intended to include the individual compounds described herein, as well as dimers, trimers and tetramers thereof which are then to be further polymerized.

In one example, the polymerizing of the monomer mixture in accordance with the present invention may be carried out at 100°C at a solids concentration of from 10 to 80 wt.%, or, preferably, from 20 to 50 wt.% of polymer solids using, for example, n-butyl acetate (bp = 126 °C) solvent. After a period of time (4-24 hr), polymerizations are chased with additional BPO, then allowed to run for another 4-10 hr.

Prior to curing or crosslinking the polymer compositions in accordance with the present invention have a weight average molecular weight of from 3,000 to 250,000 or, preferably, from 6,000 to 50,000, as determined by gel permeation chromatography (GPC) against polystyrene standards.

The polymer composition can be crosslinked via thermally induced or light induced initiation in the presence of the curing agent and a monomer containing two or more dienophile groups which are addition polymerizable, or by ring opening of the arylcyclobutene groups on the polymer in the presence of any monomer containing two or more dienophile groups.

The polymers of the present invention may be used as is or may be isolated by adding a non-solvent, such as water or methanol, to precipitate the polymer from the solution and thereafter removing the organic solvent.

The polymer compositions of the present invention may comprise one or more arylcyclobutene-containing polymers and one or more organic solvents. Suitable organic solvents are those in which the polymers are soluble. Particularly useful organic solvents are any solvents useful in the making or formulation of arylcyclobutene polymers. Exemplary organic solvents include, without limitation: aromatic hydrocarbons such as toluene, xylene, and mesitylene; alcohols such as 2-methyl-1-butanol, 4-methyl-2-pentanol, and methyl isobutyl carbinol; esters such as ethyl lactate, propylene glycol methyl ether acetate, methyl 2-hydroxyisobutyrate, methyl 3-methoxypropionate, n-butyl acetate and 3-methoxy-1-butyl acetate; lactones, such as gamma-butyrolactone; lactams such as N-methylpyrrolidinone; ethers such as propylene glycol methyl ether and dipropylene glycol dimethyl ether isomers, such as PROGLYDE^{™} DMM (The Dow Chemical Company, Midland, MI (Dow)); ketones such as cyclohexanone, 2-butanone and methylcyclohexanone; and mixtures thereof. Preferred solvents are polar protic and polar aprotic solvents.

Suitable additives that may be useful in the polymer compositions of the present invention include, without limitation, one or more of each of curing agents, crosslinkers, such as crosslinking monomers separate from the polymer, surfactants, inorganic fillers, organic fillers, plasticizers, adhesion promoters, metal passivating materials, and combinations of any of the foregoing. Suitable surfactants are well-known to those skilled in the art, and nonionic surfactants are preferred. Such surfactants may be present in an amount of from 0 to 10 g/L, and preferably from 0 to 5 g/L. Any suitable inorganic fillers may optionally be used in the present compositions, and are well-known to those skilled in the art. Exemplary inorganic fillers include, but are not limited to, silica, silicon carbide, silicon nitride, alumina, aluminum carbide, aluminum nitride, and zirconia, and mixtures thereof. The inorganic filler may be in the form of a powder, rods, spheres, or any other suitable shape. Such inorganic fillers may comprise a coupling agent, such as a silane or a titanate in conventional amounts.

Such inorganic filler may have any suitable dimensions. Inorganic filler may be used in an amount of from 0 to 80 wt%, based on the total weight of the composition. Preferably, the metal passivating material is a copper passivating agent. Suitable copper passivating agents are well known in the art and include imidazoles benzotriazoles, ethylene diamine or its salts or acid esters, and iminodiacetic acids or salts thereof.

Any crosslinkers that react with any acidic or hydrolysable functional groups on the polymer of the present invention or any olefin group or dienophile group on the polymer of the present invention may be used as crosslinkers, provided that they crosslink with the polymers of the present invention under the conditions used to cure the composition. Suitable crosslinkers include, but are not limited to, diamines, polyamines, and polythiols, including polymers having multiple amine or thiol groups, monomers having two or more olefin groups or dienophile groups, such as glycol di(meth)acrylates or diallyl phthalate, and bis-arylcyclobutene monomers wherein the cyclobutene ring contains at least one of a monovalent hydrocarbon containing group, such as a C₁ to C₆ alkyl, carboxyalkyl, keto, aldehyde, acetal, ketal, or hydroxyalkyl group. Suitable diamines may include simple aromatic diamines such as phenylene diamine, Jeffamine^{™} ethoxylated amines (Huntsman Corp., Salt Lake City, UT), or oligoimides that are capped with amine groups. The selection of such crosslinkers is within the ability of those skilled in the art.

Crosslinkers are typically used in an amount of from 0 to 20 wt.%, and preferably 0 to 10 wt.%, based on the total weight of the polymerizable monomers in the composition. The polymer compositions of the present invention find many uses, such as in photolithography, packaging, adhesive, sealing and bulk dielectric applications, such as in spin on coatings or buffer layers.

A variety of curing agents may be used in the polymer compositions of the present invention which are useful in photolithography. Suitable curing agents may aid in the curing of the polymer of the present invention, and may be activated by heat or light. Exemplary curing agents include, but are not limited to, thermally generated initiators and photoactive compounds (photogenerated initiators). The selection of such curing agents is within the ability of those skilled in the art. Preferred thermal generated initiators are free radical initiators, such as, but not limited to, azobisisobutyronitrile, dibenzoyl peroxide, and dicumylperoxide. Preferred photoactive curing agents are free radical photoinitiators available from BASF under the Irgacure brand, and diazonaphthoquinone (DNQ) compounds including sulfonate esters of a DNQ compound. Suitable DNQ compounds are any compounds having a DNQ moiety, such as a DNQ sulfonate ester moiety, and that function as photoactive compounds in the present compositions, that is, they function as dissolution inhibitors upon exposure to appropriate radiation. Suitable DNQ compounds are disclosed in U.S. Pat. Nos. 7,198,878 and 8,143,360. The amount of photoactive compound varies from 0 to 30 wt.%, based on the total weight of the polymer solids. When present, the photoactive compound is typically used in an amount of 5 to 30 wt.%, preferably from 5 to 25 wt.%, and more preferably from 10 to 25 wt.%, based on the total weight of polymer solids.

Any suitable adhesion promoter may be used in the polymer compositions of the present invention and the selection of such adhesion promoter is well within the ability of those skilled in the art. Preferred adhesion promoters are silane-containing materials or tetraalkyl titanates, and more preferably trialkoxysilane-containing materials. Exemplary adhesion promoters include, but are not limited to: bis(trialkoxysilylalkyl)benzenes such as bis(trimethoxysilylethyl)benzene; aminoalkyl trialkoxy silanes such as aminopropyl trimethoxy silane, aminopropyl triethoxy silane, and phenyl aminopropyl triethoxy silane; and other silane coupling agents, as well as mixtures of the foregoing. Particularly suitable adhesion promoters include AP 3000, AP 8000, and AP 9000S adhesion promoters (Dow Electronic Materials, Marlborough, MA).

The polymer compositions of the present invention may contain from 0 to 15 wt.% of an adhesion promoter based on the total weight of the composition, preferably from 0.5 to 10 wt.%, more preferably from 1 to 10 wt.%, yet more preferably from 2 to 10 wt.%.

The polymer compositions the present invention may be photolithographic compositions prepared by combining one or more polymers of the present invention and any organic solvents, water or additional components in any order. When the present compositions contain a curing agent such as a photoactive compound, such as a diazonaphthoquinone, an onium salt or photoinitiator, it is preferred that the curing agent is first dissolved in a suitable organic solvent or aqueous alkali, then combined with one or more present polymers and any optional surfactant, and then combined with any optional adhesion promoter. Selection of a suitable photoactive compound is within the ordinary level of skill in the art.

Any of the polymer compositions of the present invention can be used to form a layer or film suitable for use as, for example, dielectric layers, permanent bonding adhesives, or as stress buffer layers.

The polymer composition of the present invention is useful, for example, as a thin film or coating composition.

To form a thin film or coating, the polymer composition as an organic solvent solution may be drawn or coated onto a PET film and soft baked at from 90 to 125 °C for from 5 to 25 minutes prior to curing the composition.

The polymer composition or the thin film or coating composition can be crosslinked via thermally induced or light induced initiation in the presence of the curing agent initiator and, if desired a monomer containing two or more addition polymerizable groups, or by ring opening of the arylcyclobutene groups on the polymer, if desired, in the presence of any monomer containing two or more dienophile groups, or by a condensation reaction, such as between acid functional monomer groups and diamines.

In the case of a curing agent comprising a thermal or photo initiator compound or a photoactive compound, the addition polymerization curing of the polymer or coating composition can occur via initiation polymerization of the polymer, further including, if desired, a crosslinker monomer containing two or more addition polymerizable groups and at any temperature of from ambient temperature to 140 °C, for example, from 60 to 125 °C.

Curing of the polymer composition, which may or may not result in crosslinking, can also comprise heating the composition to cause ring opening thermal curing at, for example, from 170 to 230 °C, or, preferably, from 180 to 220 °C curing a crosslinker monomer containing two or more dienophile groups.

Ring opening curing refers to reaction of any addition polymerizable arylcyclobutene group-containing monomer A with any olefin or dienophile groups in the polymer composition, such as in a copolymerized monomer or in an added crosslinker monomer having two or more dienophile groups

Ring opening curing can also result in increasing the value of x in the above Structure A by ring opening of the arylcyclobutene groups in the arylcyclobutene-containing monomer A. Such ring opening can result from B-staging the polymer composition after addition polymerizing to form the polymer, thereby forming oligomers of arylcyclobutene groups.

To test the flexibility of coatings or films made from the polymer composition of the present invention, dried or soft baked films are then bent over a conical mandrel and any crack propagation is measured in distance from the base of the cone. Film flexibility can also be qualitatively observed by bending by hand, attempting to crease the film without cracking, and cutting the film with scissors to observe any chipping or cracking.

The polymer compositions of the present invention may be coated on a substrate by any suitable method. Suitable methods for disposing the present compositions include, but are not limited to, spin-coating, curtain coating, spray coating, roller coating, dip coating, vapor deposition, and lamination such as vacuum lamination, among other methods. In the semiconductor manufacturing industry, spin-coating is a preferred method to take advantage of existing equipment and processes. In spin-coating, the solids content of the composition may be adjusted, along with the spin speed, to achieve a desired thickness of the composition on the surface to which it is applied.

Typically, the polymer compositions of the present invention are spin-coated at a spin speed of 400 to 4000 rpm. The amount of the present compositions dispensed on the wafer or substrate depends on the total solids content in the composition, the desired thickness of the resulting layer, and other factors well-known to those skilled in the art. When a film or layer of the present compositions is cast, such as by spin-coating, much (or all) of the solvent evaporates during deposition of the film. Preferably, after being disposed on a surface, the composition is heated (baked) to remove any remaining solvent. Typical baking temperatures are from 90 to 130 °C, although other temperatures may be suitably used. Such soft baking to remove residual solvent is typically done for approximately 2 minutes, although longer or shorter times may suitably be used. The polymer compositions of the present invention are typically cured by heating for a period of time. Suitable curing temperatures range from 170 to 230 °C. Typically curing times range from 1 to 600 minutes.

Preferably, layers of the polymer compositions of the present invention may also be formed as a dry film and disposed on the surface of a substrate by lamination. A variety of suitable lamination techniques, including vacuum lamination techniques, may be used and are well known to those skilled in the art. In forming a dry film, the present compositions are first disposed, such as coated, onto a front surface of a suitable film support sheet such as a polyester sheet, preferably poly(ethylene terephthalate) (PET) sheet, or a polyimide sheet such as KAPTON^{™} polyimide (DuPont, Wilmington, DE), using slot-die coating, gravure printing, or another appropriate method. The composition is then soft baked at a suitable temperature, such as from 90 to 140 °C, for an appropriate time, such as from 1 to 30 minutes, to remove any solvent. A polymer film cover sheet such as polyethylene is then roll-laminated at room temperature onto the dried composition to protect the composition during storage and handling. To dispose the dried composition onto the substrate, the cover sheet is first removed. Then, the dried composition on the support sheet is laminated onto the substrate surface using roll-lamination or vacuum lamination. The lamination temperature can range from 20 to 120 °C. The support sheet is then removed (peeled), leaving the dried composition on that surface.

A wide variety of electronic device substrates may be employed in the present invention. An electronic device substrate is any substrate for use in the manufacture of any electronic device, preferably a polymer or plastic substrate. Exemplary electronic device substrates include, without limitation, semiconductor wafers, glass, sapphire, silicate materials, silicon nitride materials, silicon carbide materials, display device substrates, epoxy mold compound wafers, circuit board substrates, and thermally stable polymers. As used herein, the term "semiconductor wafer" is intended to encompass a semiconductor substrate, a semiconductor device, and various packages for various levels of interconnection, including a single-chip wafer, multiple-chip wafer, packages for various levels, substrates for light emitting diodes (LEDs), or other assemblies requiring solder connections. Semiconductor wafers, such as silicon wafers, gallium-arsenide wafers, and silicon-germanium wafers, may be patterned or unpatterned. As used herein, the term "semiconductor substrate" includes any substrate having one or more semiconductor layers or structures which include active or operable portions of semiconductor devices. The term "semiconductor substrate" is defined to mean any construction comprising semiconductive material, such as a semiconductor device. A semiconductor device refers to a semiconductor substrate upon which at least one microelectronic device has been or is being fabricated. Thermally stable polymers include, without limitation, any polymer stable to the temperatures used to cure the arylcyclobutene material, such as polyimide, for example, KAPTON^{™} polyimide (DuPont, Wilmington, DE).

When compositions of the present invention which do not contain an adhesion promoter are used, the surface of the substrate to be coated with the present compositions may optionally first be contacted with a suitable adhesion promoter or vapor treated. Such treatments improve the adhesion of the polymer compositions of the present invention to the substrate surface. Any suitable method, such as spin-coating, dip coating, spray coating, curtain coating, roll coating, or vapor deposition, may be used to contact the substrate surface with the adhesion promoter. Spin-coating is a preferred method for contacting the substrate surface with an adhesion promoter. Any suitable adhesion promoter may be used and the selection of such adhesion promoter is well within the ability of those skilled in the art. Preferred adhesion promoters are silane-containing materials, and more preferably trialkoxysilane-containing materials. Exemplary adhesion promoters useful to pretreat the substrate surface are those described above. Various vapor treatments known in the art may be used to increase the adhesion of the arylcyclobutene containing polymers of the present invention to the substrate surface, such as plasma treatments. In certain applications, it may be preferred to use an adhesion promoter to treat the substrate surface prior to coating the surface with the present compositions.

EXAMPLES: The present invention will now be described in detail in the following, non-limiting Examples:
Unless otherwise stated all temperatures are room temperature (21-23 °C) and all pressures are atmospheric pressure (∼760 mm Hg or 101 kPa).

Notwithstanding other raw materials disclosed below, the following raw materials were used in the Examples:
BCB: benzocyclobutene;
BPO: Benzoyl peroxide;
MBA: 3-methoxy butylacetate solvent;
PET: poly(ethylene terephthalate); and,
MI: maleimide.

The materials were analyzed in various disclosed ways, including, as follows:
Mole Ratio Calculation: Moles of second, third and fourth monomers to moles of arylcyclobutene-containing monomer A was calculated from the starting number of moles of arylcyclobutene monomer. The residual exotherm from B-staging was measured in J/g and compared to the total enthalpy in the bis-arylcyclobutene monomer (in J/g, for DVS it is ∼800 J/g, corresponding to 156 J/mmol of BCB). The ratio of residual exotherm to enthalpy gave a number of mmols of arylcyclobutene monomer per gram of solids; this was multiplied by the total mass in grams of solid, to give total mmols of arylcyclobutene monomer. This was compared to number of mmoles of other monomers added to give the mole ratio.
Example 1: Preparation of 4-vinyl pyridine-co-styrene co-alpha methyl-vinyl BCB: In a 100ml EasyMaxTm 402 Basic Synthesis Workstation (Mettler Toledo, Columbia, MD) reactor equipped with polytetraflouroethylene (Teflon^{™} polymer, Dupont) coated thermocouple and an overhead mechanical stirrer, a heel of n-butyl acetate (13 g) was charged with stirring. The reactor was cooled to 10 °C and purged with nitrogen for 20 minutes. In a separate 50 ml pear shaped round bottom flask, styrene (16.19 g), 4-vinyl pyridine (2.51 g), alpha methyl vinyl BCB (8.62 90% purity g), n-butyl acetate (1.78 g) and benzoyl peroxide (130 mg) were added, capped with a rubber septum and purged with nitrogen for 20 minutes. The resulting solution was drawn into a 50 ml syringe, and secured into a syringe pump. The EasyMax^{™} workstation was heated to the reaction temperature (100 °C), and then the reaction solution was added via syringe pump at a rate of 20mL/hr. After 4hr from the start of the addition, a chase of BPO in n-butyl acetate (40 mg in 7.72 g) was added via syringe. The reaction continued for a total of 8hr then was cooled to room temperature and poured into a glass jar. No further purification took place. GPC: Mn = 30.2k, Mw = 80.8k PDI= 2.68.
Example 2: Preparation of 4-vinyl pyridine-co-styrene co-n-butyl acrylate co-alpha methyl vinyl BCB: In a 100mL EasyMax^{™} 402 Basic Synthesis Workstation (Mettler Toledo) reactor equipped with a polytetraflouroethylene (Teflon^{™} polymer, DuPont) coated thermocouple and overhead mechanical stirrer, a heel of n-butyl acetate (10 g) was charged with stirring. The reactor was cooled to 10 °C and purged with nitrogen for 20 minutes. In a separate 50 mL pear shaped round bottom flask, styrene (10.30 g), 4-vinyl pyridine (2.21 g), n-butyl acrylate (7.08 g), alpha methyl vinyl BCB (7.75 g), n-butyl acetate (3.60 g) and benzoyl peroxide (110 mg) were added, capped with a rubber septum and purged with nitrogen for 20 minutes. The resulting solution was drawn into a 50 mL syringe, and secured into a syringe pump. The EasyMax^{™} reactor was heated to the reaction temperature (100 °C), and then the reaction solution was added via syringe pump at a rate of 20ml/hr. After 4hr from the start of the addition, a chase of BPO in n-butyl acetate (40 mg in 8.90 g) was added via syringe. The reaction continued for a total of 8hr then was cooled to room temperature and poured into a glass jar. No further purification took place. GPC: Mn = 31.3k, Mw = 68.9k PDI= 2.20.
Example 3: Preparation of styrene-co-n-butyl acrylate co-alpha methyl vinyl BCB: In a 100ml EasyMax^{™} 402 Basic Synthesis Workstation (Mettler Toledo) reactor equipped with a polytetraflouroethylene (Teflon^{™} polymer, DuPont) coated thermocouple and an overhead mechanical stirrer, a heel of n-butyl acetate (13 g) was charged with stirring. The reactor was cooled to 10 °C and purged with nitrogen for 20 minutes. In a separate 50 mL pear shaped round bottom flask, styrene (13.16 g), n-butyl acrylate (5.89 g), alpha methyl vinyl BCB (8.28 g), n-butyl acetate (1.97 g) and benzoyl peroxide (130 mg) were added, capped with a rubber septum and purged with nitrogen for 20 minutes. The resulting solution was drawn into a 50 mL syringe and secured into a syringe pump. The EasyMax^{™} reactor was heated to the reaction temperature (100 °C), and then the reaction solution was added via syringe pump at a rate of 20ml/hr. After 18hr from the start of the addition, a chase of BPO in n-butyl acetate (40 mg in 7.71 g) was added via syringe. The reaction continued for a total of 40hr then was cooled to room temperature and poured into a glass jar. No further purification took place. GPC: Mn = 29.9k, Mw = 71.9k PDI= 2.4.
Polymer testing: The solutions of the polymer in the indicated examples were dispensed onto poly(ethylene terephthalate) (PET) sheets and coated with a 152.4 pm (6 MIL) draw down bar to form films. The films were soft baked at 120 °C.
Mandrel Bend Test - Elongation values: The indicated soft baked polymer films on a PET substrate were bend tested on a conical mandrel and the crack distance was measured from the base in inches (cm). Elongation was determined using the ASTM D522 (2017) method and is reported in percent; an acceptable elongation value is 7% or, preferably higher.

The soft baked films were laminated onto copper coated wafers. The copper wafer laminates were cured in a nitrogen oven at 200 °C for two hours in the case of alpha methyl vinyl BCB containing copolymers and 250 °C for one hour in the case of vinyl BCB containing copolymers. Cured wafer laminates were placed in a 10 wt.% aqueous ammonium persulfate bath until the films lifted, then films were rinsed and dried and cut into strips. The resulting strips were used for DMA/TMA analysis. Polymer films were laminated onto copper clad coupons and cured for cross hatch adhesion testing as defined, below

Differential Scanning Calorimetry (DSC): To give a glass transition temperature (Tg) DSC was performed with a TA Instruments Q2000 DSC device. A sample was weighed out (2-10mg) and placed in an aluminum pan. A reference pan with no sample is placed in the reference slot of the device. The samples were equilibrated at 23 °C, then ramped linearly to 300 °C at a rate of 10 °C/minute under a nitrogen flow of 50mL/min. The reported Tg is the inflection point of the resulting DSC curve.

Thermal stability was tested by running TGA analysis (Q500TM TGA instrument, TA Instruments, New Castle, DE). A platinum 50µL pan was tared then a polymer sample (1-10mg) was loaded. The furnace is sealed and then the temperature is ramped from 25°C to 600 °C at rate of 10 °C/min under nitrogen atmosphere 25 mL/min gas flow.

Dimensional Mechanical Analysis (DMA): To give a Tg and information about modulus, DMA was measured with a TA instruments Q800. The clamp was a tension: film method. Strain was applied at 0.06% with a force of 0.1 Newtons. Each sample was a film of the dimensions 17 mm long 10 mm wide and 35 microns thick. Each sample was equilibrated at 25 °C and then ramped to 250 °C.

Thermal Mechanical Analysis (TMA): To give TMA results, a TA instruments Q400 was used. Each sample film was prepared of the dimensions of 8mm long by 5 mm wide by 35 microns thick. An initial force of 0.1 Newtons was used. Under nitrogen atmosphere, the sample was ramped to 200 °C at 10 °C/min, then cooled to -50 °C at 10 °C/min, then ramped from -50 °C to 200 °C at 10 °CC/min. On the final ramp a value for coefficient of thermal expansion (CTE) was calculated based on the dimensional change between 0 and 100 °C. An acceptable CTE is below 130 ppm/K, and a better CTE is below 90.

Cross hatch Adhesion (AKA tape test): Adhesion was measured by ASTM D3359 (2017). Each polymer film on PET was laminated onto a copper substrate, and then the PET was peeled off. The polymer film was scored in a criss-cross manner and tape (PA-280630 (51596) tape, Interpolymer Group, Canton, MA) was applied and peeled off to determine how much, if any of the film lifted from the copper surface. If no film lifts, the result is a pass. If small bits around the edges are removed, the result is a pass with minor chipping. If entire areas of film lift off on the tape, the result is a fail.

**Table 1: Polymer Film Performance**

| Example | Cross Hatch Picture | Film Roll Crack (% elongation) | DMA Tg | TMA Tg | TMA CTE (0-100 °C), ppm/K |
|---|---|---|---|---|---|
| 1 | Pass minor chip | 10% | 184 | 153 | 84 |
| 2 | Pass | 10.5% | 180 | 142 | 125 |
| 3* | Chipping | 5% | nd | 118 | 120 |

| | | | | | |
|---|---|---|---|---|---|
| * - Denotes Comparative Example; nd = not determined. | | | | | |

As shown in Table 1, above, each of the inventive polymer compositions exhibits improved flexibility and cross-hatch adhesion in comparison to the polymer composition of comparative Example 3. This is true despite the fact that the polymer of Example 3 was polymerized for 40 hr and not 8 hr as in Examples 1 and 2. Further, the polymer having a larger component of the third and fourth monomer in inventive Example 2 gave the best flexibility results. Still further, the polymer of inventive Example 1 with the highest proportion of addition polymerizable aromatic second monomers was the most heat stable.

## Claims

1. A polymer composition comprising, in copolymerized form, a monomer mixture of from 10 to 90 wt.% of one or more addition polymerizable arylcyclobutene-containing monomer A; from 5 to 70 wt.% of one or more aromatic addition polymerizable second monomers chosen from styrene, α-methyl styrene, allyloxystyrene, allyl terminated polyarylene ethers or maleimide terminated polyarylene ethers; and from 5 to 40 wt.% of one or more nitrogen heterocycle containing addition polymerizable third monomers chosen from vinyl pyridine and vinyl imidazole; wherein monomer A has Structure A: 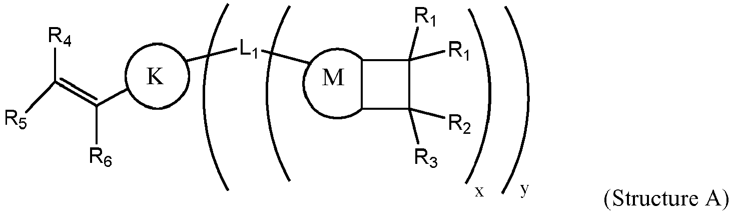 wherein K is a covalent bond or a divalent group chosen from a C₁ to C₆ alkyl substituted or unsubstituted divalent aryl group, a C₁ to C₆ alkyl substituted or unsubstituted divalent heteroaryl group, C₁₋C₃₀ divalent alkyl group, a C₁₋C₃₀ heteroatom containing alkyl group, a divalent C₁ to C₃₀ alkylene group, a carbonyl group, an ether group, a thioether group, an ester group, a carboxyl group, or a cyano group;
M is a divalent aromatic group chosen from a C₁ to C₆ alkyl substituted or unsubstituted divalent aryl group, a C₁ to C₆ alkyl substituted or unsubstituted divalent heteroaryl group;
L₁ is selected from a covalent bond or a hydrocarbon linking group having a valence of x +1, wherein;
R₁ through R₆ are each independently selected from a monovalent group chosen from hydrogen, deuterium, halogen, a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group, wherein at least one of R₁, R₂ and R₃ is chosen from a C₁ to C₆ alkyl group, a C₁ to C₆ alkoxy group, a C₁ to C₆ alkyl substituted hydrocarbon group, a heteroatom containing hydrocarbon group, a C₁ to C₆ alkyl substituted heterohydrocarbon group, a cyano group, a C₆ to C₁₂ aryl group, C₁ to C₆ alkyl substituted aryl group, a heteroaryl group, or a C₁ to C₆ alkyl substituted heteroaryl group; and
x and y are each independently an integer from 1 to 5 wherein y is 1 when L₁ is a covalent bond;
wherein all weights based on the total solids weight of monomers used to make the copolymer with all monomer wt.%s adding to 100%.

2. The polymer composition as claimed in claim 1, wherein the one or more addition polymerizable arylcyclobutene monomers A has a Structure A, wherein y is 1 and x ranges from 1 to 2.

3. The polymer composition as claimed in claim 1, wherein the amount of the one or more addition polymerizable arylcyclobutene monomers A has a Structure A wherein each of K and L₁ is a covalent bond or K and L₁ together form a C₁₋C₃₀ alkylene group or an alkyl substituted alkylene group; and at least one of R₁, R₂ and R₃ is chosen from a C₁ to C₆ alkyl group, a C₁ to C₆ alkoxy group, a C₆ to C₁₂ aryl group, C₁ to C₆ alkyl substituted aryl group, a heteroaryl group, or a C₁ to C₆ alkyl substituted heteroaryl group.

4. The polymer composition as claimed in claim 1, further comprising in copolymerized form from 5 to 40 wt.% of one or more addition polymerizable fourth monomers chosen from n-butyl acrylate, a C₈ to C₂₀ linear alkyl (meth)acrylate, or a diene monomer.

5. The polymer composition as claimed in claim 1, further comprising a separate crosslinker; a curing agent; a catalyst, or more than one of these.

6. The polymer composition as claimed in claim 1, comprising from 10 to 80 wt.% of polymer solids, based on the total weight of the polymer and the remainder of a polar aprotic or polar protic organic solvent.

7. A method of using a polymer composition to make a thin film or coating comprising:
depositing and evaporating, or coating on a substrate the polymer composition of claim 1.

## Patentansprüche

1. Polymerzusammensetzung umfassend, in copolymerisierter Form, eine Monomermischung aus 10 bis 90 Gew.-% eines oder mehrerer additionspolymerisierbarer, Arylcyclobuten enthaltender Monomere A; 5 bis 70 Gew.-% eines oder mehrerer aromatischer, additionspolymerisierbarer zweiter Monomere ausgewählt unter Styrol, α-Methylstyrol, Allyloxystyrol, allylterminierten Polyarylenethern oder maleimidterminierten Polyarylenethern; und 5 bis 40 Gew.-% eines oder mehrerer Stickstoffheterocyclen, die additionspolymerisierbare dritte Monomere ausgewählt unter Vinylpyridin und Vinylimidazol enthalten; wobei das Monomer A die Struktur A aufweist: 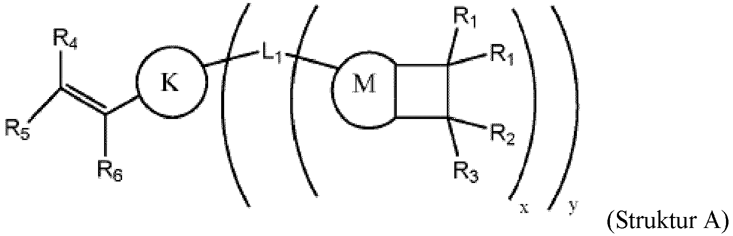
wobei K eine kovalente Bindung oder eine zweiwertige Gruppe ist ausgewählt unter einer C₁-bis C₆-Alkyl-substituierten oder unsubstituierten zweiwertigen Arylgruppe, einer C₁-bis C₆-Alkyl-substituierten oder unsubstituierten zweiwertigen Heteroarylgruppe, einer zweiwertigen C₁- bis C₃₀-Alkylgruppe, einer C₁- bis C₃₀-Heteroatom enthaltenden Alkylgruppe, einer zweiwertigen C₁- bis C₃₀-Alkylengruppe, einer Carbonylgruppe, einer Ethergruppe, einer Thioethergruppe, einer Estergruppe, einer Carboxylgruppe oder einer Cyanogruppe,
M eine zweiwertige aromatische Gruppe ist ausgewählt unter einer C₁-bis C₆-Alkyl-substituierten oder unsubstituierten zweiwertigen Arylgruppe, einer C₁-bis C₆-Alkyl-substituierten oder unsubstituierten zweiwertigen Heteroarylgruppe;
L₁ unter einer kovalenten Bindung oder einer Kohlenwasserstoffverknüpfungsgruppe ausgewählt ist, die eine Wertigkeit von x +1 aufweist, wobei:
R₁ bis R₆ jedes unabhängig ausgewählt sind aus einer einwertigen Gruppe, die ausgewählt ist unter Wasserstoff, Deuterium, Halogen, einer C₁- bis C₆-Alkylgruppe, einer C₁-bis C₆-Alkoxygruppe, einer C₁-bis C₆-Alkyl-substituierten Kohlenwasserstoffgruppe, einer Heteroatom enthaltenden Kohlenwasserstoffgruppe, einer C₁-bis C₆-Alkyl-substituierten Heterokohlenwasserstoffgruppe, einer Cyanogruppe, einer C₆- bis C₁₂-Arylgruppe, einer C₁-bis C₆-Alkyl-substituierten Arylgruppe, einer Heteroarylgruppe oder einer C₁-bis C₆-Alkyl-substituierten Heteroarylgruppe, wobei mindestens eines von R₁, R₂ und R₃ ausgewählt ist unter einer C₁-bis C₆-Alkylgruppe, einer C₁-bis C₆-Alkoxygruppe, einer C₁- bis C₆-Alkyl-substituierten Kohlenwasserstoffgruppe, einer Heteroatom enthaltenden Kohlenwasserstoffgruppe, einer C₁- bis C₆-Alkyl-substituierten Heterokohlenwasserstoffgruppe, einer Cyanogruppe, einer C₆- bis C₁₂ Arylgruppe, einer C₁-bis C₆-Alkyl-substituierten Arylgruppe, einer Heteroarylgruppe oder einer C₁-bis C₆-Alkyl-substituierten Heteroarylgruppe; und
x und y jedes unabhängig eine ganze Zahl von 1 bis 5 ist, wobei y 1 beträgt, wenn L₁ eine kovalente Bindung ist;
wobei alle Gewichte auf dem gesamten Feststoffgewicht von Monomeren basieren, die zum Herstellen des Copolymers verwendet werden, wobei alle Monomer-Gew.-% auf 100 % addieren.

2. Polymerzusammensetzung nach Anspruch 1, wobei das eine oder die mehreren additionspolymerisierbaren Arylcyclobutenmonomere A eine Struktur A aufweisen, wobei y 1 beträgt und x im Bereich von 1 bis 2 liegt.

3. Polymerzusammensetzung nach Anspruch 1, wobei die Menge des einen oder der mehreren additionspolymerisierbaren Arylcyclobutenmonomere A eine Struktur A aufweist, wobei jedes von K und L₁ eine kovalente Bindung ist oder K und L₁ zusammen eine C₁- bis C₃₀-Alkylengruppe oder eine Alkyl-substituierte Alkylengruppe bilden; und mindestens eines von R₁, R₂ und R₃ unter einer C₁- bis C₆-Alkylgruppe, einer C₁-bis C₆-Alkoxygruppe, einer C₆ bis C₁₂-Arylgruppe, einer C₁- bis C₆-Alkyl-substituierten Arylgruppe, einer Heteroarylgruppe oder einer C₁- bis C₆-Alkyl-substituierten Heteroarylgruppe ausgewählt ist.

4. Polymerzusammensetzung nach Anspruch 1, ferner, in copolymerisierter Form, 5 bis 40 Gew.-% eines oder mehrerer additionspolymerisierbarer vierter Monomere umfassend ausgewählt unter n-Butylacrylat, einem linearen C₈- bis C₂₀-Alkyl(meth)acrylat oder einem Dienmonomer.

5. Polymerzusammensetzung nach Anspruch 1, ferner einen separaten Vernetzter, ein Aushärtungsmittel, einen Katalysator oder mehr als einen/eines dieser umfassend.

6. Polymerzusammensetzung nach Anspruch 1, umfassend 10 bis 80 Gew.-% Polymerfeststoffe, auf das Gesamtgewicht des Polymers bezogen, und wobei der Rest aus einem polaren aprotischen oder polaren protischen organischen Lösungsmittel besteht.

7. Verfahren für die Verwendung einer Polymerzusammensetzung zum Herstellen eines Dünnfilms oder einer Beschichtung, umfassend: Absetzen und Verdampfen oder schichtförmiges Auftragen, auf ein Substrat, der Polymerzusammensetzung nach Anspruch 1.

## Revendications

1. Composition polymère comprenant, sous la forme copolymérisée, un mélange de monomères de 10 à 90 % en pds d'un ou plusieurs monomères A contenant de l'arylcyclobutène polymérisables par addition ; 5 à 70 % en pds d'un ou plusieurs seconds monomères aromatiques polymérisables par addition choisis parmi le styrène, l'a-méthylstyrène, l'allyloxystyrène, les polyarylène éthers terminés par un groupe allyle ou les polyarylène éthers terminés par un groupe maléimide ; et 5 à 40 % en pds d'un ou plusieurs hétérocycles azotés contenant de troisièmes monomères polymérisables par addition choisis parmi la vinylpyridine et le vinylimidazole ; dans laquelle le monomère A a la Structure A : 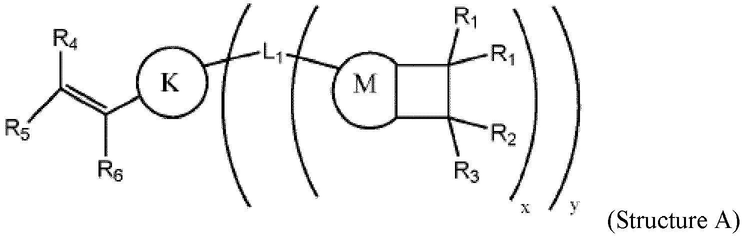
dans laquelle K est une liaison covalente ou un groupe divalent choisi parmi un groupe aryle divalent substitué ou non substitué par un groupe alkyle en C₁ à C₆, un groupe hétéroaryle divalent substitué ou non substitué par un groupe alkyle en C₁ à C₆, un groupe alkyle divalent en C₁ à C₃₀, un groupe alkyle contenant un hétéroatome en C₁ à C₃₀, un groupe alcylène divalent en C₁ à C₃₀, un groupe carbonyle, un groupe éther, un groupe thioéther, un groupe ester, un groupe carboxyle, ou un groupe cyano ;
M est un groupe aromatique divalent choisi parmi un groupe aryle divalent substitué ou non substitué par un groupe alkyle en C₁ à C₆, un groupe hétéroaryle divalent substitué ou non substitué par un groupe alkyle en C₁ à C₆ ;
L₁ est sélectionné parmi une liaison covalente ou un groupe de liaison hydrocarboné ayant une valence de x + 1 ; dans laquelle
R₁ à R₆ sont chacun indépendamment sélectionné parmi un groupe monovalent choisi parmi un hydrogène, un deutérium, un halo, un groupe alkyle en C₁ à C₆, un groupe alcoxy en C₁ à C₆, un groupe hydrocarboné substitué par un groupe alkyle en C₁ à C₆, un groupe hydrocarboné contenant un hétéroatome, un groupe hétérohydrocarboné substitué par un groupe alkyle en C₁ à C₆, un groupe cyano, un groupe aryle en C₆ à C₁₂, un groupe aryle substitué par un groupe alkyle en C₁ à C₆, un groupe hétéroaryle, ou un groupe hétéroaryle substitué par un groupe alkyle en C₁ à C₆, dans laquelle au moins l'un de R₁, R₂ et R₃ est choisi parmi un groupe alkyle en C₁ à C₆, un groupe alcoxy en C₁ à C₆, un groupe hydrocarboné substitué par un groupe alkyle en C₁ à C₆, un groupe hydrocarboné contenant un hétéroatome, un groupe hétérohydrocarboné substitué par un groupe alkyle en C₁ à C₆, un groupe cyano, un groupe aryle en C₆ à C₁₂, un groupe aryle substitué par un groupe alkyle en C₁ à C₆, un groupe hétéroaryle, ou un groupe hétéroaryle substitué par un groupe alkyle en C₁ à C₆ ; et
x et y sont chacun indépendamment un nombre entier d'une valeur de 1 à 5, y ayant la valeur de 1 lorsque L₁ est une liaison covalente ;
dans laquelle tous les poids sont basés sur les poids totaux des matières solides des monomères utilisés pour fabriquer le copolymère, tous les % en pds des monomères additionnés donnant 100 %.

2. Composition polymère telle que revendiquée selon la revendication 1, dans laquelle les un ou plusieurs monomères d'arylcyclobutène A polymérisables par addition ont une Structure A, dans laquelle y a la valeur de 1 et x varie de 1 à 2.

3. Composition polymère telle que revendiquée selon la revendication 1, dans laquelle la quantité des un ou plusieurs monomères d'arylcyclobutène A polymérisables par addition a une Structure A, dans laquelle chacun de K et L₁ est une liaison covalente ou K et L₁ conjointement forment un groupe alcylène en C₁ à C₃₀ ou un groupe alcylène substitué par un groupe alkyle ; et au moins l'un de R₁, R₂ et R₃ est choisi parmi un groupe alkyle en C₁ à C₆, un groupe alcoxy en C₁ à C₆, un groupe aryle en C₆ à C₁₂, un groupe aryle substitué par un groupe alkyle en C₁ à C₆, un groupe hétéroaryle, ou un groupe hétéroaryle substitué par un groupe alkyle en C₁ à C₆.

4. Composition polymère telle que revendiquée selon la revendication 1, comprenant en outre, sous une forme copolymérisée, de 5 à 40 % en pds d'un ou plusieurs quatrièmes monomères polymérisables par addition choisis parmi l'acrylate de *n*-butyle, un (méth)acrylate d'alkyle linéaire en C₈ à C₂₀, ou un monomère diène.

5. Composition polymère telle que revendiquée selon la revendication 1, comprenant en outre un agent de réticulation séparé ; un agent de durcissement ; un catalyseur, ou plus de l'un de ceux-ci.

6. Composition polymère telle que revendiquée selon la revendication 1, comprenant de 10 à 80 % en pds de matières solides polymères, basé sur le poids total du polymère, et le reste étant un solvant organique aprotique polaire ou protique polaire.

7. Procédé d'utilisation d'une composition polymère pour fabriquer un film fin ou un revêtement comprenant : le dépôt et l'évaporation, ou le revêtement sur un substrat de la composition polymère selon la revendication 1.
